Europäisches Patentamt

European Patent Office

Office européen des brevets

(19)

(11) Numéro de publication: **0 279 136**
**A2**

## (12) DEMANDE DE BREVET EUROPEEN

(21) Numéro de dépôt: **87402962.2**

(22) Date de dépôt: **22.12.87**

(51) Int. Cl.⁴ **A61K 7/48**

(30) Priorité: **07.01.87 FR 8700088**

(43) Date de publication de la demande:
**24.08.88 Bulletin 88/34**

(84) Etats contractants désignés:
**CH DE GB IT LI**

(71) Demandeur: **CLARINS**
**4, rue Berteaux-Dumas B.P. 174**
**F-92203 Neuilly-sur-Seine Cédex(FR)**

(72) Inventeur: **Courtin, Olivier**
**6 bis, rue de la Belle Feuille**
**F-92100 Boulogne(FR)**

(74) Mandataire: **Netter, André et al**
**Cabinet NETTER 40, rue Vignon**
**F-75009 Paris(FR)**

(54) **Cosmétique pour retarder le vieillissement de la peau et procédé d'application.**

(57) Cosmétique pour retarder le vieillissement de la peau, du type comprenant une première composition comportant au moins un principe actif hydrosoluble, sous forme de solution dans un milieu aqueux, et une deuxième composition comportant au moins un principe actif liposoluble, sous forme de solution dans un milieu huileux ou gras, lesdites compositions étant propres à être conservées séparément et à être mélangées au moment de l'application sur la peau, la concentration en au moins un principe actif dans chacune d'entre elles étant supérieure à la concentration que ce principe actif peut atteindre en émulsion.

Selon l'invention, l'une au moins des compositions contient au moins un principe actif propre à entraver le processus de vieillissement de la peau par la formation et l'action de radicaux libres.

EP 0 279 136 A2

## Cosmétique pour retarder le vieillissement de la peau et procédé d'application

Le Brevet No 84 16038 décrit un cosmétique pour retarder le vieillissement de la peau, comprenant une première composition comportant au moins un principe actif hydrosoluble, sous forme de solution dans un milieu aqueux, et une deuxième composition comportant au moins un principe actif liposoluble, sous forme de solution dans un milieu huileux ou gras, lesdites compositions étant propres à être conservées séparément et à être mélangées au moment de l'application sur la peau, la concentration en au moins un principe actif dans chacune d'entre elles étant supérieure à la concentration que ce principe actif peut atteindre en émulsion.

Le but de la présente invention est de conférer à un cosmétique de ce genre des fonctions que ne présentaient pas les produits décrits dans le Brevet précité.

L'un des principaux processus de dégradation dans le temps des tissus cutanés comporte la formation de radicaux libres à partir des éléments de ces tissus (fibres, cellules, enzymes) sous diverses influences, notamment sous l'action de radiations extérieures telles que la lumière naturelle. Les radicaux libres attaquent à leur tour les tissus cutanés par des réactions en chaine qui se poursuivent tant qu'existe un radical libre, c'est-à-dire tant que celui-ci ne s'est pas combiné avec un groupement chimique donnant naissance à une molécule stable.

Ce rôle de capteur de radicaux libres est normalement joué par des enzymes présentes dans le tissu cutané, notamment la superoxyde dismutase et la catalase. Dans certaines circonstances, le rôle de ces enzymes n'est pas suffisant pour bloquer totalement l'action destructrice des radicaux libres.

L'action négative de ces radicaux libres au niveau du tégument cutané se traduit par une attaque des membranes cellulaires, une destruction de l'ADN cellulaire, une dégradation des fibres du tissu conjonctif (collagène, élastine) assurant à la peau sa fermeté et sa jeunesse

L'invention propose d'introduire dans l'une au moins des compositions d'un cosmétique selon le Brevet No 84 16038 au moins un principe actif propre à entraver le processus de vieillis sement de la peau résultant de la formation et de l'action de radicaux libres.

Les substances ainsi mises en oeuvre peuvent agir selon différentes modalités
- Elles peuvent agir directement en tant que bloqueuses de radicaux libres Il s'agit alors de molécules possédant des propriétés anti-oxydantes.
-Elles peuvent stimuler les enzymes bloquant les radicaux libres. - Enfin, elles peuvent maintenir l'intégrité du film hydrolipidique autoprotecteur contre les radiations génératrices de radicaux libres.

Les propriétés ci-dessus ne sont cependant pas toujours suffisantes pour qu'une substance soit, dans un excipient donné, active contre le développement et l'action biologique dévastatrice des radicaux libres. En effet, le dosage à une juste valeur est essentiel pour l'obtention de l'efficacité recherchée, une concentration excédentaire pouvant conduire à un effet inverse de celui recherché. De plus, il faut tenir compte des conditions de toxicité et de tolérance.

Selon l'invention, la première composition, ou solution aqueuse, peut comporter en particulier l'un au moins des principes actifs hydrosolubles suivants :
- des composés riches en phosphore, notamment sous la forme d'extrait de pollen; le phosphore permet à la peau une rétention de calcium, qui est un oligo-élément favorisant l'activité des enzymes qui bloquent les radicaux libres;
- la vitamine C, notamment sous forme d'extrait végétal de fructus acerola (cerisier des Antilles); la vitamine C est un anti-oxydant;
- les oligo-éléments zinc, cuivre, magnésium, fer, manganèse, notamment sous forme d'une solution aqueuse d'origine naturelle, cofacteurs favorisant l'activité des enzymes naturelles contenues dans la peau et déjà citées pour leur rôle anti-radicaux libres.

La seconde composition, ou solution huileuse, comporte en particulier l'un au moins des éléments liposolubles suivants :
- la vitamine E présentant des propriétés anti-oxydantes, notamment sous forme d'un mélange de tocophérols extraits d'huiles végétales (telles qu'huile de soja) et purifiés, et/ou d'huile de germes de blé et/ou d'huile de pépins de framboises, ces deux dernières huiles étant naturellement riches en tocophérols;
- le gamma-oryzanol, provenant notamment de l'huile de son de riz; cette molécule présente une activité normalisatrice du film hydrolipidique cutané naturel qui protège les cellules épidermiques contre les agents agressifs environnants et notamment contre les radiations génératrices de radicaux libres.

La composition huileuse comporte en outre avantageusement l'un au moins des éléments suivants, qui n'ont pas d'influence directe sur le processus de vieillissement par les radicaux libres, mais qui exercent des fonctions anti-vieillissement en coopération avec les éléments ci-dessus :
- l'acide gamma-linolénique, notamment sous forme d'huile de bourrache ou d'huile végétale de sisymbrium irio; cette molécule possède le pouvoir de renforcer les mécanismes de défense naturelle de la cellule, vraisemblablement par une activation enzymatique, et la propriété de faciliter la pénétration des principes actifs associés vers les couches profondes de l'épiderme;

- des substances biologiques jouant un rôle dans la métamorphose des insectes, notamment l'huile de bombyx mori extraite du corps de la chrysalide du papillon; la métamorphose est une opération métabolique très importante qui met en oeuvre des substances biologiques extrêmement actives; en extrayant ces substances actives au moment de la métamorphose, on obtient une réactivation des processus de fonctionnement des cellules vivantes.

Le produit cosmétique selon l'invention contient avantageusement, outre les éléments énumérés ci-dessus, tout ou partie de ceux mentionnés dans le Brevet No 84 16038, pour l'obtention d'une activité anti-vieillissement plus complète.

L'utilisation d'un cosmétique tel qu'il vient d'être défini peut se faire soit en appliquant successivement sur la peau la composition aqueuse puis la composition huileuse, ou inversement, soit en mélangeant les deux compositions sur les surfaces internes des mains et en appliquant sur la peau l'émulsion obtenue, comme décrit dans le Certificat d'Addition No 85 02518

On donne ci-après un exemple de cosmétique selon l'invention.

Ce cosmétique comprend une composition aqueuse et une composition huileuse contenues dans des récipients distincts.

Ces compositions contiennent les principes actifs et extraits naturels suivants, dans les concentrations pondérales indiquées.

## Composition aqueuse

Elle contient, en solution dans un gel aqueux :
- Manuronate de silanol    3%
- Extrait biologique de rate bovine    5%
- Extrait biologique de moelle    5%
- Silymarine    2%
- PCA Na    5%
- Panthénol    0,5%
- Mucopolysaccharides    1,5%
- Acides aminés végétaux    2%
- Extrait végétal d'échinacée    5%
- Extrait de pollen    3%
- Extrait de fructus acerola    2%

## Composition huileuse

Elle contient, en solution dans une huile absorbable par l'épiderme :
- Insaponifiables d'avocat Soja-Karité    3%
- Beurre de Pendadesma    1%
- Huile de noix    5%
- Tocophérols naturels    3%
- Huile de germes de blé    3%
- Huile de pépins de framboises    3%
- Huile de bourrache    5%
- Gamma oryzanol    0,5%
- Huile de sisumbrium irio    2%
- Huile de bombyx mori    1%

On trouve dans cet exemple les mêmes constituants que dans l'exemple du Brevet No 84 16038, qui exercent les fonctions décrites dans ce Brevet. S'y ajoutent les constituants dont les propriétés ont été décrites ci-dessus, pour fournir un cosmétique présentant une action très complète contre le vieillissement de la peau.

Outre son role mentionné plus haut dans la prévention de la formation des radicaux libres, l'extrait de pollen exerce également une fonction de nutrition de la peau. Le pollen est une association naturelle d'acides aminés (notamment arginine, lysine et acide glutamique), de vitamines (particulièrement du groupe B), de glucides, de phosphore.

Le pollen, qui est l'aliment azoté de l'abeille, combine un ensemble de facteurs nutritifs assimilables par les cellules cutanées.

L'huile de germes de blé participe également à la nutrition cellulaire par l'apport de vitamines et de triglycérides.

Quant à l'huile de sisymbrium irio, plante asiatique dont on presse les graines, elle participe, avec l'huile de chrysalide de bombyx mori, à une action de régénération par stimulation des mécanismes du développement cellulaire. Cette huile est utilisée par les indigénes des pays de récolte pour leurs soins de beauté.

Le cosmétique décrit à titre d'exemple peut bien entendu être modifié sans sortir du cadre de l'invention. En particulier, d'autres constituants que ceux mentionnés peuvent y être ajoutés pour élargir encore son action. Certains constituants peuvent au contraire être supprimés si on souhaite une action plus spécialisée. Enfin, certains constituants peuvent être remplacés par d'autres constituants exerçant les mêmes fonctions. Par exemple, l'huile de bourrache peut être remplacée par l'huiles de primevère du soir ou huile d'onagre, contenant également de l'acide gamma-linolénique, quoiqu'en quantité moindre.

## Revendications

1.-Cosmétique pour retarder le vieillissement de la peau, comprenant, selon l'une des revendications 1 à 9 du Brevet No 84 16038, une première composition comportant au moins un principe actif hydrosoluble, sous forme de solution dans un milieu aqueux, et une deuxième composition comportant au moins un principe actif liposoluble, sous forme de solution dans un milieu huileux ou gras, lesdites compositions étant propres à être conservées séparément et à être mélangées au moment de l'application sur la peau, la concentration en au moins un principe actif dans chacune d'entre elles étant supérieure à la concentration que ce principe actif peut atteindre en émulsion, caractérisé en ce que l'une au moins des compositions contient au moins un principe actif propre à entraver le processus de vieillissement de la peau par la formation et l'action de radicaux libres.

2.-Cosmétique selon la revendication 1, caractérisé en ce qu'il contient au moins un principe actif ayant des propriétés anti-oxydantes.

3.-Cosmétique selon l'une des revendications 1 et 2, caractérisé en ce qu,il contient au moins un principe actif propre à stimuler les enzymes bloquant les radicaux libres.

4.-Cosmétique selon l'une des revendications précédentes, caractérisé en ce qu'il contient au moins un principe actif propre à maintenir l'intégrité du film hydrolipidique autoprotecteur contre les radiations génératrices de radicaux libres.

5.-Cosmétique selon l'une des revendications 2 à 4, caractérisé en ce que la première composition comporte au moins un principe actif choisi dans le groupe formé par les composés riches en phosphore, la vitamine C et les composés riches en oligo-éléments.

6.-Cosmétique selon la revendication 5, caractérisé en ce que la première composition comporte au moins un extrait naturel choisi dans le groupe formé par un extrait de pollen riche en phosphore, un extrait végétal de fructus acerola et une solution d'oligo-éléments d'origine naturelle.

7.-Cosmétique selon la revendication 5, caractérisé en ce que la première composition comporte tous les extraits énumérés dans la revendication 6.

8.-Cosmétique selon l'une des revendications précédentes, caractérisé en ce que la seconde composition comporte au moins un principe actif choisi dans le groupe formé par la vitamine E, l'acide gamma-linolénique, le gamma-oryzanol et les substances biologiques jouant un rôle dans la métamorphose des insectes.

9.-Cosmétique selon la revendication 8, caractérisé en ce que la seconde composition comporte au moins un extrait naturel choisi dans le groupe formé par un mélange de tocophérols extraits d'huiles végétales et purifiés, l'huile de germes de blé, l'huile de pépins de framboises, l'huile de bourrache, le gamma-oryzanol extrait d'huile de son de riz, l'huile végétale de sisymbrium irio et l'huile de bombyx mori.

10.-Cosmétique selon la revendication 8, caractérisé en ce que la seconde composition comporte tous les extraits énumérés dans la revendication 9.

11.-Procédé d'application d'un cosmétique selon l'une des revendications précédentes, caractérisé en ce qu'on applique successivement les deux compositions sur la peau, dans un ordre ou dans l'autre.

12.-Procédé d'application d'un cosmétique selon l'une des revendications 1 à 10, caractérisé en ce qu'on mélange les deux compositions sur les surfaces internes des mains et qu'on applique sur la peau l'émulsion obtenue.